# EUROPEAN PATENT APPLICATION

(11) **EP 3 988 165 A1**
(43) Date of publication of application: **27.04.2022**
(21) Application number: 21203983.8
(22) Date of filing: 21.10.2021
(51) Int. Cl.: A61N 2/02

(54) **MAGNETIC FIELD THERAPY DEVICE FOR PELVIC FLOOR STIMULATION**

(30) Priority: 21.10.2020 EP 20203040
(71) Applicant: Storz Medical AG, 8274 Tägerwilen (CH)
(72) Inventor: NOVAK, Pavel, 8234 Stetten (CH); Schulz, Manfred, 8274 Tägerwilen (CH)
(74) Representative: Lohr, Jöstingmeier & Partner

(57) **Abstract**

A magnetic field therapy system configured for pelvic floor stimulation comprising an electrical pulse generator, at least one coil and at least one support body. The support body including a soft and/or flexible material forming a hollow space configured for holding the coil.

## Description

### Field of the invention

The invention relates to a magnetic field therapy device and a coil therefor, which improves pelvic floor stimulation.

### Description of the related art

A magnetic field therapy device includes an electrical pulse generator connected to a coil. The coil is configured to generate a pulsed magnetic field by the electrical pulses from the pulse generator.

A coil including multiple windings together with a fluid cooling system is disclosed in WO 2017/212253 A1. Due to the complex structure, such coils are comparatively thick and have a solid and rigid structure. For a pelvic floor stimulation, coils may be embedded into a chair, as disclosed in EP 3 479 872 A1. The disadvantage is, that such a chair is large, expensive and can hardly be moved. US2019/0030357 A1 discloses a transcranial magnetic stimulator. In which the circuit includes at least two inductance coils, a direct current, a charging and discharging circuit and a controller. The transcranial magnetic stimulator includes a pillow base and the transcranial magnetic stimulation circuit. A containing cavity is disposed in the pillow base. The inductance coils are disposed in the containing cavity and their central lines of the inductance coils are coincided with each other.

### Summary of the invention

The problem to be solved by the invention is to provide a magnetic field therapy device which is portable, easy to use and provides no discomfort to a person using the device. Further, a good alignment between a magnetic field coil and a person's pelvic floor.

Solutions of the problem are described in the independent claims. The dependent claims relate to further improvements of the invention.

A first embodiment includes a support body which may have an outer shell further having a hollow space. The hollow space holds a coil for a magnetic field therapy device which may be configured for pelvic floor stimulation. The coil is a specific magnetic field therapy coil. The coil may include at least one winding of a wire, which may be completely embedded into the support body. The coil and/or the wire of the coil may be flexible. A flexible wire increases the comfort of the device, by causing the coil to adapt to the contour of the person. Additionally, a flexible wire makes the alignment between a magnetic field coil and a person's pelvic floor easier. The embedded wire prevents a displacement of the coil, such that the device is even more comfortable and an easy alignment may be ensured. Furthermore, the embedded wire may minimize the free space in the device and thereby minimizing the sources of contamination and making the sanitation of the device more convenient.

The support body may include a soft and/or flexible material. A person to which the magnetic field therapy is applied may sit on the body, such that the coil in the hollow space of the body is located under the person. The coil may be located in close proximity to the pelvic floor of the person, such that magnetic field of the coil may interact with the body of the person and it may interact specifically with the pelvic floor of the person.

Immediately sitting on the coil is very uncomfortable, as the coil normally has a comparatively hard structure due to the thick copper wires inside the coil and the housing of the coil. Further, most coils have a diameter in a range between 10 and 20 cm and a thickness between 2 and 4 cm which is very difficult to sit on. A person trying to sit on the coil would rather slide to its side.

The support body may have a size large enough for a person to sit on. It may have a length and a width or a diameter comparable to a chair. There may be different sizes matching to different persons. The height of the support body with a coil inserted into the hollow space may be larger than the thickness of the coil, although the thickness of the support body without a coil may be less than the thickness of the coil.

A support body may have a cylindrical or toroidal shape with a diameter between 20 cm and 50 cm, a typical diameter may be 35 cm. A height may be between 4 cm and 10 cm with a typical height of 5 cm. The body may also have a cuboid shape with length and width between 20 cm and 50 cm, a typical length and width may be 35 cm. A height may be between 4 cm and 10 cm with a typical height of 5 cm.

The support body may have a skin to hold and/or protect its interior. The support body may be inflatable by air or a gas. The support body may include a top part and a bottom part. At least one part, the top part and/or the bottom part, may include a soft and/or flexible material. The flexible material may be cellular foam or foamed plastic. The flexible material may adapt to the contour of the person, making it more comfortable. The bottom part may include stiffer material than the top part, preferably a stiffer cellular foam and/or a solid plastic material and/or a metal. The stiffer material may increase the stability of the device and increase the comfortability. Additionally, a stiffer bottom part improves the handling of the device, since alignment of the coil may be easier.

The support body may also include a reinforcement, which may include a stiffer cellular foam and/or a solid plastic material. The reinforcement may be in the top part and/or the bottom part. The support body may include means for holding the coil within the hollow space at a predetermined position. The coil may be fixed or held in the support body, preferably at a predetermined position. It may be glued into the support body. Preferably, the coil may be completely embedded in a flexible material, which may be a cellular foam or foamed plastic. The hollow space may be filled with the flexible material, thereby completely embedding the coil. Thus, the coil and the support body may form a single piece. The support body may be a selaed unit and/or it may seal the coil against the environment. The deposit of sources of infections such as germs and/or other sources of contamination, as well as debris or other unwanted particles may be minimized by completely embedding the coil. Furthermore, the device may be easier to sanitize, since any cavities may be minimized. These advantages may even more increased if the support body and the coil form a single piece.The support body may have at least one alignment structure for aligning a person and/or the person's pelvic floor with the support body and/or the coil. Such an alignment structure may be an anatomically formed support structure. It may be a support structure at a rear side of the support body configure to prevent a person sitting on the support body from sliding backwards from the support body. There may also be at least one elongated centering structure which may prevent a person sitting on the support body from sliding sidewards from the support body. There may also be an alignment structure having a shape like a bike saddle on which a person may sit. The alignment structures provided herein may improve alignment and therefore improve the effects of the magnetic field to the person and/or the region of the person's body like the pelvic floor which should receive the magnetic field.

The hollow space of the support body may be adapted to receive a plurality of different coils, wherein normally only one coil may be located in the hollow space. The hollow space may have a connection to the outside of the hollow space which may allow to insert a coil from the outside. This connection me be a channel to the outside. The channel may be narrower than the largest size of the coil, which may be at the winding section of the coil. The channel may be extended to the required size. The hollow body may be filled with a flexible material, such as such cellular foam or foamed plastic, thereby completely embedding the coil. Preferably, the coil and the support body may form a single piece. In another embodiment, the coil is fixed or held in the support body, preferably at a predetermined position. It may be glued into the support body. Alternatively, it may be held by at least one of a clamp, a locks or a Velcro strip (hook-and-loop fastener).

In an embodiment, the body may have a removable cover, sheet or pillowcase, which may be removed for cleaning.

In an embodiment, a magnetic field therapy device includes at least one coil and at least one support body. The magnetic field therapy device may include a magnetic field therapy coil which is a handheld coil having a hard housing and a handle. The magnetic field therapy device may also include a magnetic field therapy coil which further includes at least one winding of a flexible wire which is completely embedded into the support body. Connection to a generator may be provided by an external cable. Such a magnetic field therapy coil may not have a housing and/or a handle.

A magnetic field therapy system may include magnetic field therapy device and an electrical pulse generator.

### Description of Drawings

In the following the invention will be described by way of example, without limitation of the general inventive concept, on examples of embodiment with reference to the drawings.
- Figure 1: shows a sectional view from the top of a first embodiment.
- Figure 2: shows a side view of the first embodiment.
- Figure 3: shows a top view of the first embodiment.
- Figure 4: shows a top view of a coil.
- Figure 5: shows a side view of the coil.
- Figure 6: shows a sectional view from the top of a second embodiment.
- Figure 7: shows a top view on a circular support body.
- Figure 8: shows an embodiment with additional support structures in a sectional side view.
- Figure 9: shows a front view of a support body.
- Figure 10: shows a further embodiment of the support body.
- Figure 11: shows an embodiment with a coil centering.
- Figure 12: shows an embodiment with reinforcements.
- Figure 13: shows a magnetic field therapy device.
- Figure 14: shows another embodiment in a sectional side view.
- Figure 15: shows another embodiment in a sectional top view.
- Figure 16: shows a coil which is completely sealed by the material of the support body.
- Figure 17: shows a coil with a cooling pipe which is completely sealed by the material of the support body.

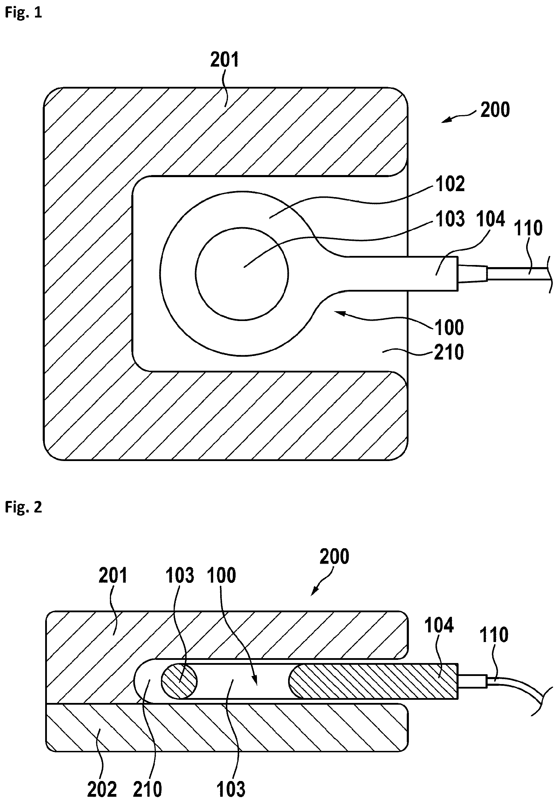

In Figure 1, a sectional view from the top of a first embodiment is shown. The sectional view shows the top part 201 of the support body 200. The support body 200 may include some soft and/or elastic material, includes a hollow space 210 which is holding a coil 100 being part of a magnetic field therapy device. The hollow space 210 may be filled with a flexible material, embedding the coil 100, such that the coil 100 and the support body 200 may form a single piece. The coil 100 may include a winding section 102, which holds at least one and preferably a larger number of windings. The winding section may also include cooling means, for example liquid cooling means. The windings may be flexible.

At the center of the winding section, there may be a central opening 103. The coil also may work when there is no such central opening. The coil may further have a handle 104, which simplifies handling of the coil. For electrical contact and supplying the currents through the windings of the coil, a cable 110 may be provided. The coil is located in a hollow space 210 of support body 200, which may be filled with a flexible material. The coil may be completely embedded in said flexible material.

In Figure 2, a side view of the first embodiment is shown. In this embodiment, the support body 200 has a cuboid shape, where the contour of the hollow space 210 is also cuboid. The support body 200 has a top part 201 and a bottom part 202. The top part may include some soft and/or elastic material, while the bottom part may include a stiffer material. The edges may be rounded or chamfered. In this Figure, a person receiving the magnetic field of the coil may sit on top of the support body, whereas the bottom side of the support body may be placed on a chair or any other surface. In this embodiment, the support body has a roughly symmetrical shape, such that top and bottom part may be exchanged. There may be other embodiments, where top and bottom are different.

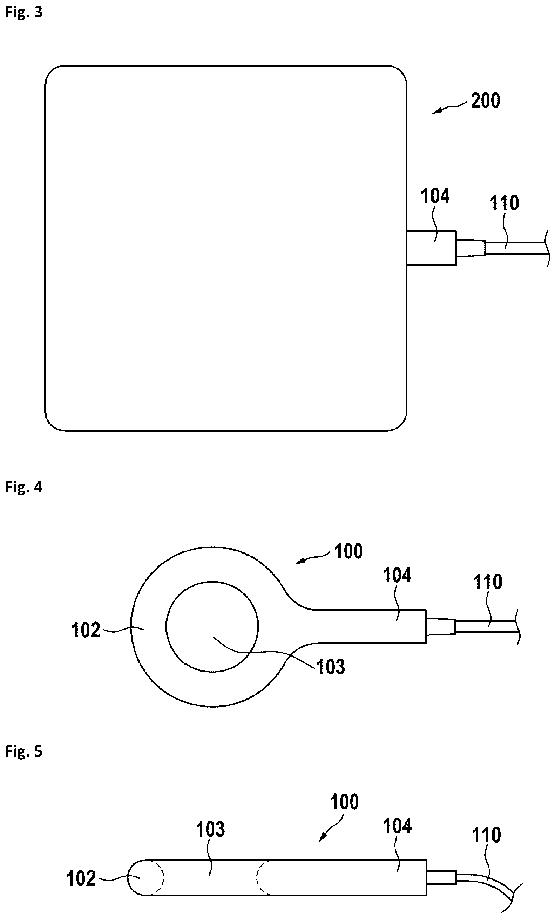

In Figure 3, a top view of the first embodiment is shown. The support body 200 may provide a planar surface on which a person may sit.

In Figure 4, a top view of a coil 100 are shown. The coil 100 has a winding section 102 which may enclose a central opening 103. A handle 104 may be provided at the winding section to simplify a handling of the coil. A cable 110 which may extend from the handle may be provided to supply current for generating magnetic fields in the coil.

In Figure 5, a side view of the coil 100 is shown. The coil may have a comparatively flat shape, but due to its lateral extensions, it would not be comfortable for a person sitting on the coil. Therefore, the support body 200 provides an enlarged surface of softer material to allow comfortable sitting.

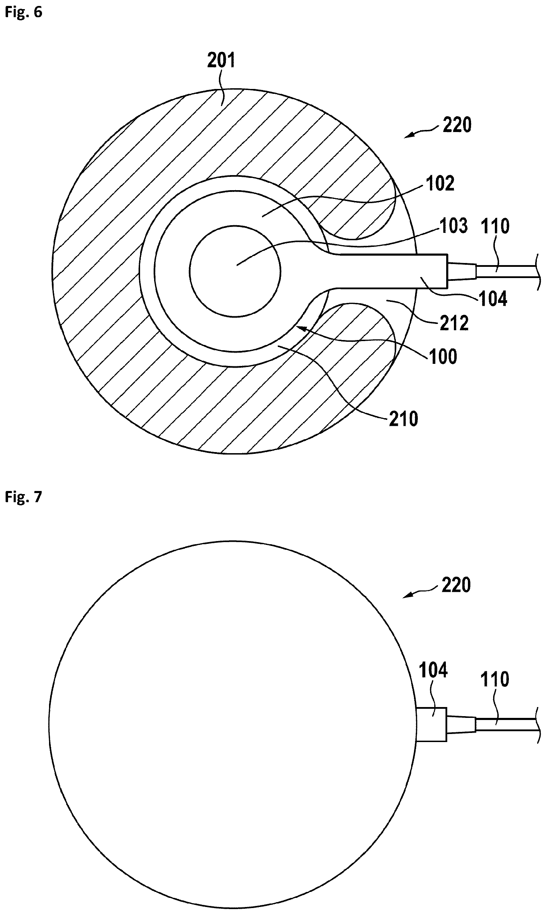

In Figure 6, a sectional view from the top 201 of a second embodiment is shown. In this embodiment, the support body 220 has a cylindrical or toroid shape. The basic elements are the same as in the first embodiment with a modification in the hollow space 210. Here, the hollow space has a channel 212 to the outside which holds the support body 200 at the coil 100. The channel may further prevent debris or other unwanted particles to penetrate into the hollow space. The coil 100 may still be removed or another coil may be inserted into the hollow space of the support body, as the channel enlarges due to the elasticity of the material of the support body and allows the coil to move through the channel. The first embodiment as mentioned above may also be provided with such a channel. Further, the embodiment shown in here may also be provided without a channel. Also, the coil 100 may be completely embedded in a flexible material, filling the hollow space 210. Such that the coil 100 and the support 200 body may form a single piece. In the case of a toroid shape, there may be a hole through the center of the support body 200.

In Figure 7, a top view on a circular support body 220 of the previous Figure is shown. A sectional side view of this second embodiment would be the same as shown in Figure 2 of the first embodiment.

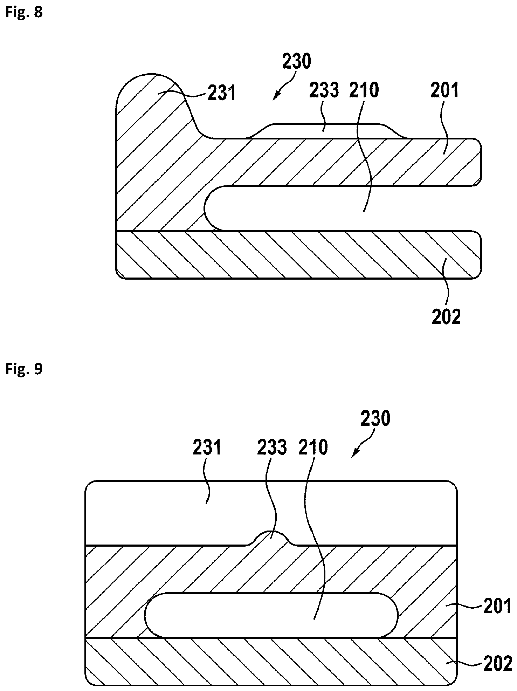

In Figure 8, an embodiment with additional support structures in a sectional side view. A rear support 231 may be provided to prevent a person sitting on the support body to slip backwards over the edge of the support body. Further, a centering means 233 may be provided to center the person sitting on the support body 230. The rear support 231 and the centering means 233 may be used individually or in conjunction. They may provide an improved centering of a person sitting on the support body with respect to the coil. It may also be possible to add support structures like the rear support 231 at the sides of the support body.

In Figure 9, a front view of the previous support body is shown. Here also, the rear support 231 and the centering means 233 are shown.

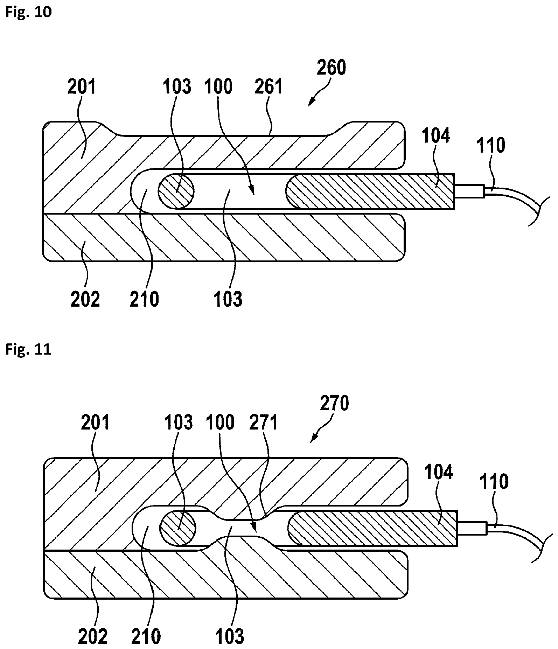

In Figure 10, a further embodiment of a support body 260 is shown. Here, a groove or recess is provided in the top surface which may make seating more comfortable and may further a centering of the person sitting on the support body.

In Figure 11, an embodiment with a coil centering device is shown. Here, a support body 270 includes at least one coil centering means 271 which may be a protrusion into the hollow space located at a position where the central opening 103 of the coil should be located. Such a centering means allows a more precise adjustment of the relative position between the body or a part of the body of the person to receive a magnetic field, like a pelvic floor with respect to the coil. The channel 212 as shown in Figure 6 may also provide at least partially such a centering.

Herein, the term centering is used in conjunction with adjusting a desired position of the coil with respect to the body or part of the body to receive a magnetic field. Coil centering means 271 may also be combined with other means for holding the coil within the hollow space. Such means may for example be a channel 212 as shown previously, and/or clips, clamps, locks, or Velcro strips or embedding the coil in a flexible material. The space 110 may be filled with a filler which also may be a foam.

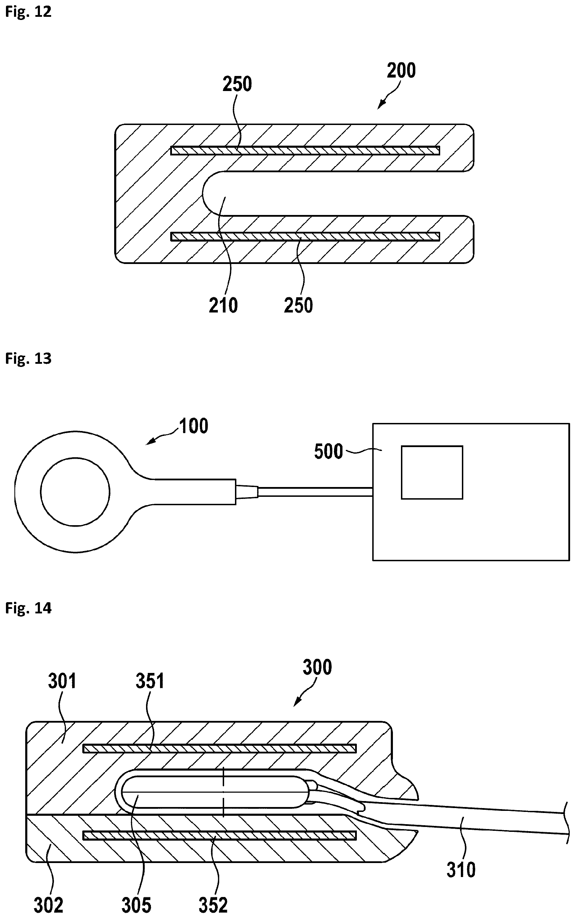

In Figure 12, an embodiment with reinforcements 250 is shown. Such a reinforcement may include stiffer foam and/or a solid plastic material, like a plastic plate or more complex structures. Such reinforcement structures may improve comfort of the person sitting on the support body and they may also provide protection of the coil within the support body. There may be only one reinforcement, e.g. a reinforcement on top of the coil. If there are two reinforcements, they may be different by material and/or size.

In Figure 13, a magnetic field therapy device is shown, which includes an electrical pulse generator 500 and a coil 100 connected by the cable 110 to the electrical pulse generator.

Figure 14 shows another embodiment in a sectional side view. A support body 300 similar as described above may hold a coil 305. Here, the coil 305 has no housing (hard shell) and therefore may have some degree of flexibility. The coil may include litz wires 306 which has good electrical properties for high frequency pulses and is comparatively flexible. There may be a flexible cooling pipe 307 wound around the litz wires 306, which may conduct a cooling liquid like water to cool the coil. Accordingly, this embodiment is more flexible and comfortable to sit on as the previous embodiment, which may use a standard coil with a hard housing. Further, in this embodiment, the coil may not need a handle. Instead, a comparatively flexible cable may provide the electrical connection from a pulse generator to the coil. As the coil itself has no housing, the support body may take over the housing function, such that it completely encloses the coil. The support body may closely fit to the coil to prevent the coil from slipping out of the body. There may be a zipper or any other means for opening the support body to gain access to the coil. The support body may be lockable, such that only qualified service personnel may gain access to the coil.

Further, there may be at least one first reinforcement 351 and/or at least one second reinforcement 352. These reinforcements may have similar properties and functions as described above. As the coil is more flexible, the first and/or the second reinforcement may also provide some degree of flexibility. As the coil may provide some heat, at least one reinforcement may have thermal conductive properties to distribute the heat of the coil over a larger area. Such a reinforcement may include a sheet or plate of metal.

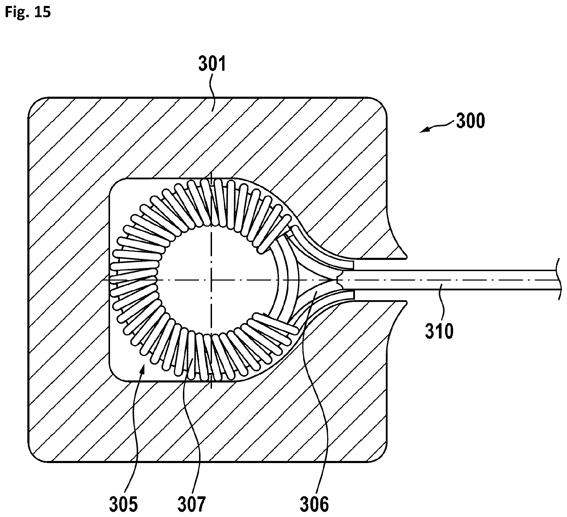

Figure 15 shows the previous embodiment in a sectional top view.

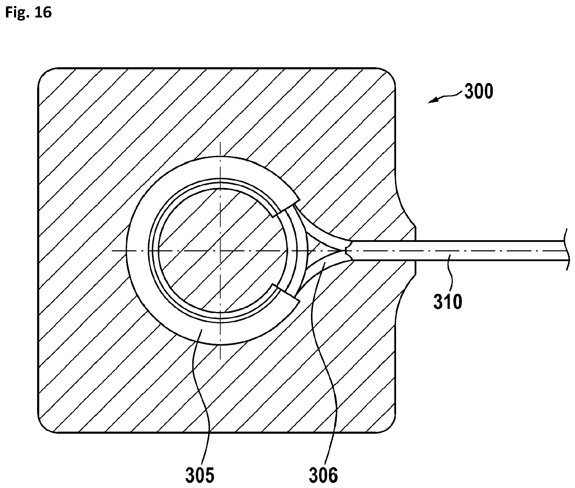

Figure 16 shows a coil 305 which is completely sealed by the material of the support body 300.

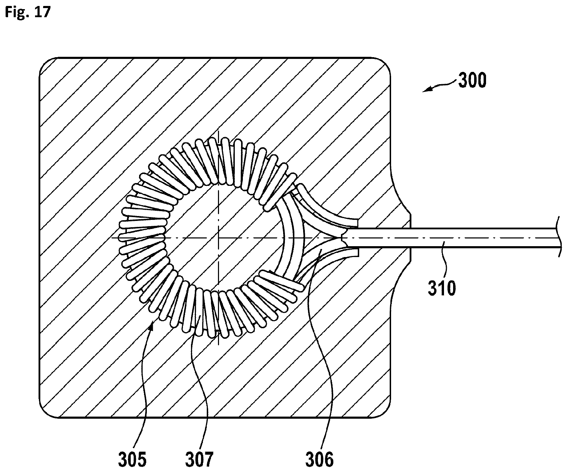

Figure 17 shows a coil 305 with a cooling pipe 307 which is completely sealed by the material of the support body 300.

### List of reference numerals

- 100: coil
- 102: winding section
- 103: central opening
- 104: handle
- 110: cable
- 200: support body
- 201: top part
- 202: bottom part
- 210: hollow space
- 212: channel to the outside
- 220: circular support body
- 230: anatomical support body
- 231: rear support
- 233: centering means
- 250: reinforcement
- 260: support body with recess
- 261: recess
- 270: support body with coil centering means
- 271: coil centering means
- 300: support body
- 301: top part
- 302: bottom part
- 305: coil without housing
- 306: wire
- 307: cooling pipe
- 310: cable
- 351: first reinforcement
- 352: second reinforcement
- 500: electrical pulse generator

## Claims

1. A support body for a coil of a magnetic field therapy device configured for pelvic floor stimulation, including a soft and/or flexible material forming a hollow space holding the coil,**characterized in, that** the coil includes at least one winding of a flexible wire, which is completely embedded into the support body.

2. A support body according to claim 1
**characterized in, that**
the coil is flexible.

3. A support body according to any of the previous claims,
**characterized in, that**
the support body comprises an top part and a bottom part, wherein at least one part comprises a cellular foam or foamed plastic.

4. A support body according to any of the previous claims,
**characterized in, that**
the bottom part comprises a stiffer cellular foam and/or a solid plastic material and/or a metal.

5. A support body according to any of the previous claims,
**characterized in, that**
the support body comprises means for holding the coil within the hollow space at a predetermined position.

6. A support body according to any of the previous claims
**characterized in, that**
the coil is completely embedded in a flexible material.

7. A support body according to any of the previous claim,
**characterized in, that**
the support body forms a single piece and/or support body forms a single piece together with the coil and/or the support body completely encloses the coil and seals the coil against the environment.

8. A support body according to any of the previous claims,
**characterized in, that**
the support body comprises a skin to hold and/or protect its interior.

9. A support body according to any of the previous claims,
**characterized in, that**
the support body comprises a reinforcement including a stiffer cellular foam and/or a solid plastic material and/or a metal.

10. A support body according to the previous claim,
**characterized in, that**
the reinforcement including a material with heat conducting properties.

11. A support body according to any of the previous claims,
**characterized in, that**
the support body comprises at least one alignment structure for aligning a person and/or the person's pelvic floor with the support body and/or the coil.

12. A support body according to any of the previous claims,
**characterized in, that**
the support body comprises at least one support structure at a rear side of the support body configure to prevent a person sitting on the support body from sliding backwards from the support body.

13. A support body according to any of the previous claims,
**characterized in, that**
the support body comprises at least one elongated centering structure which may prevent a person sitting on the support body from sliding sidewards from the support body.

14. A magnetic field therapy device includes at least one support body according to any of the previous claims and a magnetic field therapy coil.

15. A magnetic field therapy system comprising an electrical pulse generator, at least one coil and at least one support body according to any of the previous claims.
